# EUROPEAN PATENT SPECIFICATION

(11) **EP 4 419 152 B1**
(45) Date of publication and mention of the grant of the patent: **18.11.2026**
(21) Application number: 22801230.8
(22) Date of filing: 17.10.2022
(51) Int. Cl.: A61L 2/18, A01N 59/00, B08B 17/02, C01F 1/00, F24F 5/00, F28C 1/00

(54) **USE OF A COMPOSITION COMPRISING PERFORMIC ACID AS BIOCIDE IN INDUSTRIAL COOLING SYSTEMS**
VERWENDUNG EINER PERAMEISENSÄURE ENTHALTENDEN ZUSAMMENSETZUNG ALS BIOZID IN INDUSTRIELLEN KÜHLSYSTEMEN
UTILISATION D'UNE COMPOSITION COMPRENANT DE L'ACIDE PERFORMIQUE EN TANT QUE BIOCIDE DANS DES SYSTÈMES DE REFROIDISSEMENT INDUSTRIELS

(30) Priority: 21.10.2021 IT 202100027020
(43) Date of publication of application: 28.08.2024
(73) Proprietor: Chimec S.p.A., 00144 Roma (IT)
(72) Inventor: BUCCOLINI, Marco, 62032 Camerino MC (IT); MANTARRO, Milena, 00128 Rome (IT); CIARDI, Umberto, 03025 Monte San Giovanni Campano FR (IT); ZOCCARATO, Tatiana, 00040 Lanuvio RM (IT); BANCONE, Michele, 00045 Genzano di Roma RM (IT); BRUNO, Massimo, 00071 Pomezia RM (IT); VOLPONI, Andrea, 00132 Rome (IT)
(74) Representative: Montemurro, Riccardo
(86) International application number: PCT/IB2022/059932
(87) International publication number: WO 2023/067470

(56) References cited:
- US-A1- 2017 295 784
- US-A1- 2019 092 661

## Description

### FIELD OF THE INVENTION

The present invention relates to the use of a composition comprising performic acid as biocide and/or to hinder the regrowth of microorganisms in an industrial cooling system, such as for example, a cooling tower.

### STATE OF ART

All industrial plants are characterized by the generation of more or less important heat emissions in quantitative terms: however, the generated "residual" heat, except few cases, has a low energy content which does not make the recovery possible or convenient. In such context, therefore, it appears necessary to adopt effective solutions allowing an adequate dissipation of the generated heat. Among the different solutions available to this purpose there are the industrial cooling systems and, in particular, the cooling towers. The cooling towers consist in devices inside thereof the forced evaporation of a minimum amount of water takes place, with respect to the main mass, by subtracting heat from the mass itself which, then, cools down (evaporation latent heat).

A system of the "cooling tower" type, in particular, provides the presence of feed water (MU), a fraction of evaporated water (VAP) and a fraction of water expelled from the system (BD). The system, then, tends to concentrate the existing water (number of concentration NC) as well as the chemical and biological species soluble and/or vehiculated in the fluid.

The operating conditions used in an industrial cooling tower are often so as to favour the growth of microorganisms with following danger of "biological risk" in the plant, formation of biofilms and generation of corrosion phenomena. In particular, the above-described water concentration phenomenon, together with a bad management of the BD fraction and the use of persistent chemical products can often lead to an accumulation of such products inside the cooling system.

These particular conditions then often make necessary the treatment of the industrial cooling systems with biocidal products. The use of these product in cooling systems however is frequently associated to a series of problems, thereamong, in particular, the possibility that the reaction by-products of the used biocidal substance could act as nutrients for the treated microorganisms, then by favouring microbial *regrowth* phenomena, particularly relevant inside a semi-closed environment, such as a cooling tower.

An increase in the microbial load in a cooling tower - due to the *regrowth* phenomenon - can often favour the formation of biofilms within the system, that is the formation, promoted by hydrolysis processes, of a gelatinous layer around the cells of some microorganisms. The biofilm partially consists of cells, microbiological components and organic and inorganic material present in water; the polymeric nature of some components confers the biofilm a significant consistence and resistance to external agents.

As a consequence of the biofilm formation, the microorganisms can fix on the surface of the treated cooling systems, by making the industrial water disinfection process particularly difficult. Under such conditions, the water flow of the cooling towers, in fact, results to be often insufficient to determine a complete removal of the microbial biofilm which has formed.

As mentioned above, one of the main problems associated to the biofilm formation inside industrial cooling systems is represented by corrosion. In particular, inside the biofilm protected layer, the microorganisms can trigger a quick deterioration of the walls of the cooling towers and of the thermal exchange systems through the formation of corrosive by-products. Not only the microbiological reactions can accelerate the corrosion reactions, but it is also important to consider that the microorganisms present in the biofilm can accelerate the oxygen consumption. This can translate into a progressive localized lack of oxygen, which can trigger, in some microorganisms, a transition to a fermentative metabolism and the production of organic and inorganic acids (hydrogen sulfide), with a consequent pH decrease. Among the different microorganisms, the anaerobic bacteria can form, under such conditions, corrosive by-products.

Under such conditions, the biofilm which has formed can even hinder the action of anticorrosive products, by inhibiting them from reaching the surfaces of the treated systems.

A second problem associated to the formation of biofilms in the industrial cooling systems is represented by a high risk of blocking the lines in the system and/or of reducing the effectiveness of thermal exchange: the biofilm, in fact, generates an insulating layer on the thermal exchange systems, which can induce a decrease in the system effectiveness.

Apart from what already indicated, an additional critical issue which can be associated to the formation of biofilms in cooling systems is represented by the possibility of increasing the risk of dangerous microbiological contaminations, for example of *Legionella pneumophila. Legionella pneumophila* is a ubiquitous bacterium which can be found in natural aqueous environments such as lakes and rivers, but even in the groundwater, sea water or environments generally characterized by the presence of water. The bacterium low concentration in its natural environments generally does not lead to the disease (Legionellosis) and even less to the generation of outbreaks. The probability that a source of *Legionella pneumophila* could cause an infection depends upon the bacterial count, upon the way in which it multiplies and upon the possibility to form aerosol. The generation of aerosol of water contaminated by *Legionella,* in fact, is associated to the possibility of transmitting the disease.

As already mentioned, the cooling effect of the water circulating in a cooling tower takes place by evaporation of the same during the contact with the air crossing through the tower. The air flow generated in a cooling tower then can drag therewith water droplets potentially contaminated by bacteria such as *Legionella.* Consequently, the dispersion of such aerosol can represent a mechanism for spreading *Legionella* in the environment.

Among the biocides commonly used within the treatment of industrial plants there is the performic acid (PFA). It is known that PFA is a highly reactive, instable, substance whose biocidal capability then can be strongly influenced by the environmental conditions and, in particular, by temperature. However, the use of PFA as biocide generally is limited in the field to the treatment of industrial or civil waste water whose quality results to be extremely different with respect to the water present in an industrial cooling system.

Contrary to the industrial cooling systems, in fact, the waste water results to be less - if not at all - subjected to problems linked to possible *regrowth* phenomena. In other terms, in waste water there is a reduced risk of forming biofilms with relative structural consequences: this because in the systems for treating waste water the used operating conditions (for example, the flow speed, the water quality and the type of materials) do not favour the formation of biofilms. Or rather, contrary to what happens in industrial cooling systems, in the waste water *regrowth* phenomena of not pathogen microorganisms can favour the biomass degradation and then the decrease in parameters such as the biochemical oxygen demand (BOD) and the chemical oxygen demand (COD), with a consequent increase in the quality of the discharged flow.

In particular, apart from being chemically controlled and managed according to the regulations in force, the waste water is usually vehiculated through specific channels. The waste water temperature, then, depends upon the season and the region and the channels can have sizes ranging from few meters to kilometres. Therefore, phenomena of water concentration do not appear and along the whole path this is generally in equilibrium with the environmental air. Consequently, the plants vehiculating the waste water are so as to make improbable the dispersion of pathogen microorganisms, such as *Legionella,* through the formation of aerosol. This, differently with respect to a system of the "cooling tower" type, makes almost null the probability of spreading the pathogen by aerosol. US 2017/295784 A1 and US 2019/092661 A1 disclose compositions to use as biocide in industrial cooling systems.

The phenomena of water concentration typical of an industrial cooling system and the consequent temperature variation even in a sensitive and sudden way depending upon the involved plant region, up to now have discouraged in the field the use of compositions comprising PFA with biocidal purpose.

Considering the above-discussed particularly relevant differences with respect to the waste water treatment systems, the capability of developing new products appears then to be of primary importance, which could exert an effective biocidal action even under the specific environmental conditions and structural features of the industrial cooling systems, so as to be able to contrast microbic *regrowth* phenomena inside such systems.

### SUMMARY OF THE INVENTION

The object of the present invention is then to provide the use of a composition acting effectively as biocide and/or allowing to hinder the regrowth of microorganisms inside an industrial cooling system.

Such problem is solved by the use of a composition according to claim 1 and a method according to claim 8. . Preferred features of the present invention are set forth in the depending claims.

The invention in particular provides the use of a composition comprising performic acid (PFA) as biocide, as well as a method providing the use of such composition and/or to hinder the regrowth of microorganisms inside an industrial cooling system, for example a cooling tower.

Contrary to what suggested up to now in the field, the authors of the present invention have found that PFA is well suited to be used in the treatment of industrial cooling systems (for example, cooling towers) since, apart from exerting an effective biocidal action even under temperature conditions different from the environmental ones typical of waste water and even if in presence of high concentrations of dissolved oxygen, surprisingly allows to hinder the onset of microbial *regrowth* phenomena in such systems.

Advantageously, as clearly revealed by the results of laboratory tests shown in the experimental section, the inventors have demonstrated that both PFA and the by-products of its decomposition, such as the formate ion, do not act as nutrients in industrial cooling systems, and then do not favour microbial *regrowth* phenomena in the treated systems.

The inventors have further found that PFA is particularly effective in contrasting the microbial *regrowth* phenomena in industrial cooling systems when used in a concentration comprised between 0.5 and 0.8 ppm.

The possibility of using effectively PFA in the range of concentrations detected by the inventors with the purpose of contrasting the microbial regrowth in the above-mentioned systems, results to be particularly advantageous since higher dosages of PFA could involve high values of COD of water, corrosion problems due to peroxides or a too low pH conditioning.

Therefore, the invention relates to:
- the use of a composition comprising performic acid (PFA) as biocide and/or to hinder the regrowth of microorganisms in an industrial cooling system according to claim 1; and
- a method to prevent, reduce or remove and/or to hinder the regrowth of microorganisms in an industrial cooling system, according to claim 8.
Other advantages and features of the present invention will result evident from the following detailed description.

### BRIEF DESCRIPTION OF FIGURES

**Figure 1****.** Graphs illustrating the corrosion rate of carbon steel (left) and 316L stainless steel (right) exposed to PFA, PAA, or PPA. The error bars relate to the standard error.
**Figure 2****.** Results of the tests performed on 28/04 and 30/04 (on diluted water). From left: White 28/04; Solution 1 (28/04) and Solution 2 (28/04). All samples reveal a microbial load of at least 10⁶ UFC. By continuing, the white 28/04 diluted and inoculated on 30/04, Solution 1 diluted on 30/04 and Solution 2 diluted on 30/04.
**Figure 3****.** Results of the tests performed on 06/05. On the left, the instructions for reading the easy cults with the contamination examples. The samples, respectively, represent: From left: White 06/05;
Solution 1: 10ppm of Formic acid (06/05) and Solution 2:_50ppm of Formic acid (06/05). All samples reveal a microbial load of at least 10⁶ UFC.
**Figure 4****.** Results of the tests performed on 06/05. Regrowth verification. The samples of figure 3 were read every 48 hours. The samples do not reveal regrowth phenomena since the bacterial count is equivalent to that of the white (approximately 10⁶ UFC).
**Figure 5****.** Results of the tests in presence of hydrogen peroxide - Regrowth verification. From top downwards the vials inoculated with:
   - Not treated water_Positive 7/8 vials;
   - Water treated with 30ppm of Formic acid_Positive 7/8 vials;
   - Water treated with 10ppm of Formic acid_Positive 7/8 vials;
   - Water treated with 30ppm of Formic acid and 23,4 ppm of Hydrogen peroxide_Positive 5/8 vials;
   - Water treated with 10ppm of Formic acid and 7,8 ppm of Hydrogen peroxide_Positive 6/8 vials
   are shown.

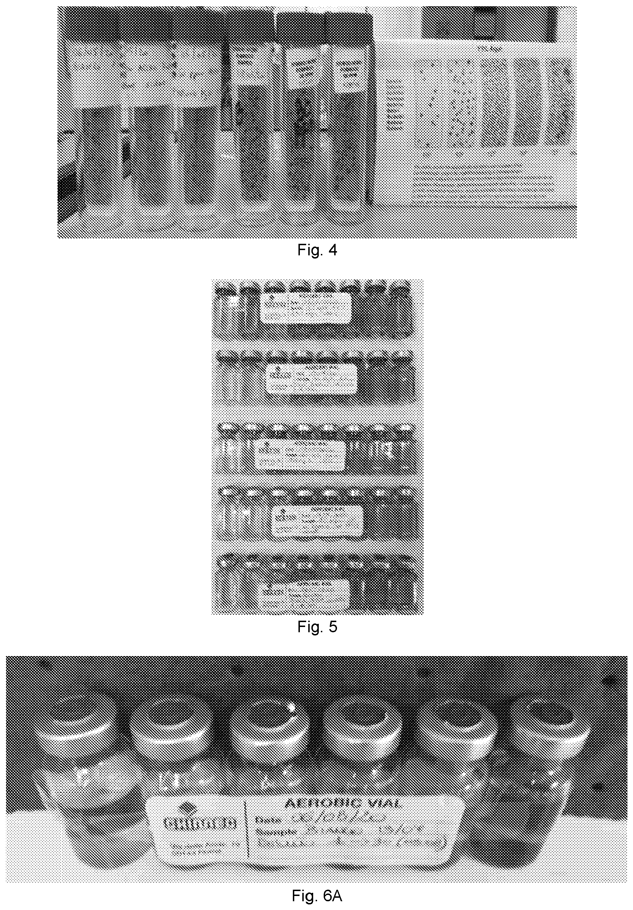

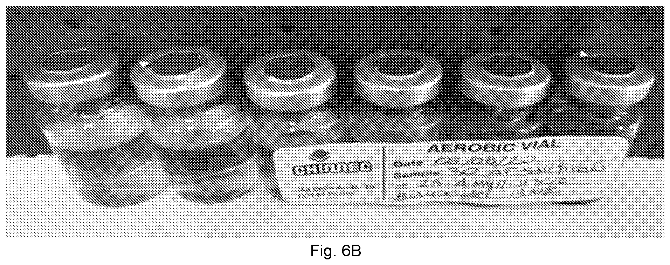

Figure 6. Results of the tests in presence of hydrogen peroxide and Formate ion - Regrowth verification.
   - Figure 6A: Vials inoculated on 06/08 with not treated water_Positive 5/6 vials
   - Figure 6b: Vials inoculated on 06/08 with water treated with 30 mg/L Solution Salified Formic Acid + 23,4 mg/L H₂O₂. Positive 5/6 vials.

### DETAILED DESCRIPTION

The terms used in the present disclosure are as generally understood by the person skilled in the art, unless otherwise indicated.

As previously mentioned, the present disclosure firstly relates to the use of a composition comprising performic acid (PFA) as biocide and/or to hinder the regrowth of microorganisms in an industrial cooling system.

The performic acid (also known as peroxiformic acid or PFA) is a chemical compound with formula HCOOOH, a strongly oxidating organic acid due to tendency to release oxygen by reducing in formic acid.

The term "biocidal", used in the context of the present disclosure relates to the capability of a composition according to any one of the herein described embodiments to prevent, reduce, inhibit and/or interrupt the growth of microorganisms. In other terms, the term "biocidal" comprises the capability of a composition according to the disclosure to reduce, remove or inactivate contaminants of microbial type inside an industrial cooling system. The term "biocidal" can be used interchangeably with the terms "disinfectant" or "antimicrobial".

A composition comprising PFA as biocide according to the disclosure is a composition capable of exerting an antimicrobial activity, for example antibacterial, antifungal, antiviral or anti-parasitic activity, in particular capable of exerting an activity aimed at contrasting the formation of biofilms. The antimicrobial activity further comprises activities capable of preventing the deterioration of a surface within a treated industrial cooling system.

The term "microorganisms" as used in the context of the present disclosure comprises Gram-positive and/or Gram-negative bacteria, fungi, viruses, spores, parasites. Preferably, the compositions comprising PFA according to any one of the herein described embodiments are particularly effective as biocides and/or to hinder the regrowth of Gram-positive and/or Gram-negative bacteria such as bacteria belonging to the genus *Enterococcus, Escherichia, Legionella, Klebsiella, Citrobacter, Pseudomonas, Staphylococcus, Bacillus, Clostridia.* Preferably, said compositions result to be effective against the species *Escherichia coli, Legionella pneumophila, Staphylococcus aureus, Bacillus subtilis, Klebsiella pneumoniae, Enterobacter sp., Serratia sp., Desulfovibrio sp.*

The compositions comprising PFA according to the disclosure result to be effective against yeast species such as Saccharomyces cerevisiae and Candida albicans, filamentous fungi such as Aspergillus niger, algae such as Chlorella vulgaris, Euglena gracilis and Selenastrum capricornutum, and other analogous microorganisms and unicellular organisms (for example phytoplankton and protozoa).

The term "to reduce" used in the context of the present disclosure in particular relates to the capability of reducing the number of microorganisms in the treated system. Typically, the obtained amount of microbial reduction can be calculated in terms of log reduction, log₁₀. A composition according to any one of the herein described embodiments is a composition preferably capable of providing a microbial reduction so as to obtain an expected value, in terms of log₁₀, of 3.

The presence of microorganisms in an industrial cooling system can be detected or determined quantitatively by means of any one of the techniques known to a person skilled in the art within microbiology and analytical chemistry.

As mentioned, the authors of the present invention have found that the products of PFA degradation, for example the formic acid, do not act as nutrients for the microorganisms in the treated cooling systems. Surprisingly then the use of a composition comprising PFA according to the disclosure in an industrial cooling system, allows to hinder the formation of nutrients which could act as substrate for the regrowth of microorganisms in the previously treated systems. Under the expression "to hinder the *regrowth",* in the context of the present disclosure then the capability of a composition according to the disclosure is meant to act as not favouring the microbial regrowth thanks to the inability of PFA or degradation products thereof to act as nutrient and/or substrate for the development of microorganisms. A preferred embodiment according to the present disclosure relates to the use of a composition comprising PFA to hinder the regrowth of microorganisms in an industrial cooling system within the subsequent 12-48 hours from the treatment of the same with the above-mentioned composition.

A composition comprising PFA suitable to be used according to the present disclosure is a composition comprising PFA in an amount effective as biocide and/or to hinder the regrowth of the microorganisms of interest in the considered industrial cooling system. Under the expression "effective amount" an amount is meant sufficient to reduce, inhibit the growth and/or to hinder the regrowth of the wished microorganisms above and/or inside a surface or substrate of the considered cooling system, for example inside cooling water.

A preferred aspect of the disclosure in particular relates to the use of a composition according to any one of the herein described variants, comprising a concentration of PFA between 0.5 ppm and 10 ppm. The measurement unit "ppm" is defined as "parts per million" and relates to an amount of PFA in a composition as such or with a value obtained after said composition has reached the equilibrium.

An additional aspect of the disclosure relates to the use of a composition comprising a concentration of PFA between 0.5 and 5 ppm, between 0.5 and 2 ppm, between 0.5 and 1 ppm, between 0.5 and 0.8 ppm. Still more preferably, PFA is present in a composition according to the invention in a concentration equal to 0.5 ppm, 0.8 ppm, 1 ppm, or 2 ppm.

A composition comprising PFA according to any one of the herein described variants can further comprise hydrogen peroxide, H₂O₂.

The amount of hydrogen peroxide inside a composition according to any one of the herein described embodiments preferably is so as not to influence negatively the biocidal capability of the used composition as well as its capability of hindering the regrowth of microorganisms. The hydrogen peroxide usable inside a composition according to any one of the herein described embodiments can be in any form, for example even under the form of a substance which is capable of generating hydrogen peroxide once in contact with an aqueous solution. An example of substance which is capable of generating hydrogen peroxide, for example, is a precursor compound of hydrogen peroxide such as sodium percarbonate or sodium perborate.
According to an aspect of the disclosure the hydrogen peroxide is present in a composition according to the invention in excess with respect to the concentration of PFA. Preferably, the ratio between PFA and hydrogen peroxide in a composition according to the invention, meant as molar ratio, is comprised between 10:1 and 1:10, still more preferably it is comprised between 1:1 and 1:4.

The PFA present in any one of the herein described compositions can even be generated *in situ* starting from hydrogen peroxide and formic acid according to any one of the methods known to a person skilled in the art. According to an aspect of the disclosure a composition according to any one of the herein described embodiments is a solution in equilibrium further comprising formic acid and hydrogen peroxide.

As it is known, the reaction between formic acid and hydrogen peroxide to form PFA can be catalysed by acid under controlled temperature conditions. The compositions comprising PFA according to any one of the previously described embodiments then can further comprise a catalyst. Examples of catalysts which can be used inside a composition according to the disclosure comprise organic or inorganic acid catalysts, such as sulfuric acid, hydrochloric acid and/or a mixture thereof.

For the PFA formation reactions catalysed by acid, the speed with which the composition comprising the reagents and PFA reaches the equilibrium can depend upon several factors, for example the concentrations of the reagents, the reaction temperature, and/or the concentration of the catalysing agent, such as for example a strong organic or inorganic acid.

Preferably, in order to generate in situ PFA, the formic acid reagent can be present in a composition according to the disclosure in an amount comprised between 50-75% by weight with respect to the total weight of the composition. Still to generate in situ PFA, the hydrogen peroxide reagent can be present in a composition according to the disclosure in an amount comprised between 30-50% by weight with respect to the total weight of the composition.

Alternatively, any substance which is capable of generating performic acid can be used in a composition according to the present disclosure Such substance for example can be a formate salt, for example sodium formate, or a formate ester. According to an embodiment, the formates can be provided in solid form, for example as formate starch.

An embodiment of the disclosure in particular relates to the use of a composition comprising PFA, formic acid and hydrogen peroxide, wherein the formic acid and the performic acid are in an amount comprised between 2 and 25% by weight with respect to the total weight of the composition, whereas the molar ratio between the PFA and hydrogen peroxide is comprised between 10:1 and 1:10, preferably between 1:1 and 1:4.

According to an aspect of the disclosure the composition comprising PFA according to any one of the previously described embodiments can be obtained *in situ* starting from mixing a first composition comprising formic acid with a second composition comprising hydrogen peroxide for the time sufficient to produce PFA in the concentrations effective to act as biocide and/or to hinder the regrowth of microorganisms in the treated system.

According to an additional aspect of the disclosure said composition comprising PFA can be obtained in situ starting from mixing a first composition comprising a substance capable of generating formic acid with a second composition comprising a substance capable of generating hydrogen peroxide in any one of the previously described embodiments.

According to an aspect of the disclosure an amount equal to approximately 1 ppm of PFA is generated within less than one minute, within less than two minutes, within less than 5 minutes, within less than 10 minutes from mixing formic acid and hydrogen peroxide in a composition of the invention.

Preferably, the compositions according to the present disclosure comprise a catalyst in a concentration comprised between 0.0001 and 20 % by weight with respect to the total weight of the composition, preferably in the range 1-10% by weight.

The compositions comprising PFA according to any one of the herein described embodiments are preferably in form of aqueous solution, or alternatively in form of aqueous suspension. The compositions according to the present disclosure then can contain water in amounts which depend upon the modes or techniques with which the composition is obtained or subsequently applied. The water preferably acts as solvent of PFA and of possible further components of the composition of the invention.

The compositions comprising PFA according to any one of the herein described embodiments can further include one or more additive agents such as diluents, stabilizing agents, anticorrosive compounds, surfactants, adjuvants. The compositions according to the disclosure can include, for example, additives capable of improving/increasing the biocidal activity of the compositions and/or capable of imparting additional properties to the same, such as, for example, a cleaning, anti-limescale and/or anti-corrosive capability.

The additives present in a composition comprising PFA according to the present disclosure in any case have to be additives compatible with PFA and with the other active ingredients possibly present in the composition, so as to preserve the biocidal activity thereof as well as the stability over time.

Examples of stabilizing agents which can be used in a composition according to the present disclosure are compounds which are capable of reducing the possibility that PFA or in case the hydrogen peroxide degrade inside the composition, by forming oxygen and not oxidized species.

It is preferable that the compositions comprising PFA according to the present disclosure are applied in an industrial cooling system which requires them, immediately after or in a short time range after the preparation of the same.

The herein described compositions comprising PFA can be used as such as biocide and/or to hinder the regrowth of microorganisms in an industrial cooling system or, alternatively, they can be diluted before use by using any suitable diluting agent such as for example, water.

As previously mentioned, a composition according to any one of the herein described embodiments can be used as biocide and/or to hinder the regrowth of microorganisms inside and/or above any environment, component, substrate or surface characterizing an industrial cooling system.

Industrial cooling systems that can be treated with a composition according to the present disclosure comprise direct flow cooling systems, closed cycle cooling systems, air washers, cooling basins and pasteurizers.

A particular example of industrial cooling system which is well suitable to be treated with a composition according to any one of the herein described embodiments is a system of the "cooling tower" type. As previously mentioned, under "cooling tower", in the context of the present disclosure a device of an industrial plant is meant, inside thereof the forced evaporation of a minimum water amount takes place, with respect to the main mass, by subtracting heat from the mass itself which, then, cools down.

According to an aspect of the present disclosure a composition according to any one of the herein described embodiments can be used as biocide and/or to hinder the regrowth of microorganisms present on a surface or substrate of the considered industrial cooling plant (for example, walls, floors, pipes), on a specific surface of the used equipment (for example process instruments, production instruments, hard surfaces) or inside cooling water.

In an aspect of the disclosure a cooling tower and/or means contained inside thereof (for example, filling/packing materials) are put in contact with a composition comprising PFA according to any one of the herein described embodiments.

Preferably, the compositions according to any one of the herein described embodiments are used as biocide and/or to hinder the regrowth of microorganisms inside cooling water of an industrial cooling system.

The compositions of the disclosure are well suitable to be used for treating cooling water characterized by an amount of dissolved oxygen comprised between 2 and 20 ppm. Preferably, the cooling water the treatment with composition according to the present disclosure relates to is characterized by a pH comprised between 5 and 9.5, and/or by a temperature comprised between 5 and 50°C.

As a person skilled in art knows, the water used as come *makeup* (MU) in a cooling tower can have different chemical-physical features depending upon the source. The possible sources of cooling water suitable to be treated with a composition according to the invention comprise osmotic water, river water, lake water or sea water depending upon the geographic area in which the system is present.

According to an aspect of the present disclosure the compositions according to any one of the herein described embodiments are particularly suitable for treating cooling water characterized by a one or more of the properties shown in the following table 1:

**Table 1 - Typical parameters of makeup water of cooling towers, considering the possible water sources**

| **Parameter** | **Concentration range** | **Measurement unit** |
|---|---|---|
| **pH** | 5.0-9.5 | - |
| **Conductivity** | 0 - 5 | S/m |
| **Calcic Hardness** | 0 - 800 | mg/l_{CaCO3} |
| **Total Hardness** | 0-800 | mg/l_{CaCO3} |
| **Soluble Iron** | 0-5 | mg/l |
| **Orto Phosphates** | 0-10 | mg/l |
| **Chlorides** | 0- 20 | g/l |
| **Sulfates** | 0 - 2 | g/l |
| **Turbidity** | 0-50 | NTU |
| **Silicates** | 0- 100 | mg/l |
| **Alkalinity P** | 0- 1000 | mg/l_{CaCO3} |
| **Alkalinity T** | 0-1000 | mg/l_{CaCO3} |
| **TSS (Total suspended solid)** | 0 - 200 | mg/l |

The concentration of the analytes in water shown in Table 1 can even be increased depending upon the number of cycles (NC) of the cooling tower from 1.2 times up to 5 times.

In the tower and/or evaporating condenser cooling circuits, the disposal of the thermal load mainly takes place by evaporation of a water portion; as consequence of the evaporation phenomena, the salts contained in water tend to concentrate. How much the Salts concentrate is function of NC. The concentration of the salts is adjusted by means of the purge determining the number of cycles thereof.

The compositions according to any one of the previously described embodiments can be introduced inside a cooling water flow requiring the treatment, for a sufficient period of time so that the composition could perform the biocidal action as well as it could hinder the regrowth of microorganisms in the treated water.

By pure way of example, the compositions comprising PFA according to the disclosure can be introduced in a cooling water flow for a time range from about 30 minutes to about 4 hours at the wished concentration according to any one of the previously illustrated embodiments, optionally according to one or more subsequent applications.

According to an aspect of the present disclosure the herein described compositions can be introduced inside cooling water on a periodical basis. The compositions comprising PFA according to the disclosure can be for example dosed according to an application regime suitable to prevent, reduce, inhibit the growth and/or to hinder the regrowth of microorganisms.

As used herein, the expression "suitable application regime" comprises the definition of a suitable time range between different applications of a composition comprising PFA according to the present disclosure By pure way of example, a suitable application regime for example is once a week, once every two days or still at least once a day.

According to an aspect of the disclosure a suitable application regime of a composition comprising PFA according to the disclosure comprises a time range between one application and another one comprised between 2 and 10 hours between an application and the following one, or between 3 to 5 hours.

The duration of each application and the time range between one application and another one could be defined by a person skilled in the art based upon the type and amount of microorganisms the treatment relates to as well as based upon the involved type of environment, substrate, component of the cooling system.

The present disclosure also relates to a method to prevent, reduce or remove and/or to hinder the regrowth of microorganisms in an industrial cooling system, comprising at least the following step of:
a) putting into contact said cooling system with a composition comprising performic acid (PFA) in an amount effective as biocide and/or to hinder the regrowth of said microorganisms.

According to an aspect of the present disclosure in the herein described method, the composition comprising PFA is in a form according to any one of the previously described embodiments. A preferred embodiment of the method according to the present disclosure provides that the PFA is generated in situ starting from formic acid and hydrogen peroxide according to any one of the modes known to a person skilled in the art, such as for example illustrated previously.

Preferably, said composition comprises PFA in a concentration comprised between 0.5 and 10 ppm, preferably between 0.5 and 2 ppm, still more preferably between 0.5 and 1 ppm.

The step a) of the herein described method, according to an embodiment, provides to put in contact a composition comprising PFA according to any one of the previously described variants with a substrate or a surface of the cooling system and/or directly with cooling water requiring it.

According to an aspect of the disclosure the herein described method provides the use of a device generating a composition comprising PFA according to any one of the herein described embodiments which is capable of vehiculating the so-generated composition at the section, substrate or surface of the cooling system requiring the treatment.

According to an aspect of the disclosure the step a) of the herein described method preferably has a duration at least equal to 30 minutes. It is further preferable that the step a) is performed at a temperature comprised between 5 and 50°C.

Herebelow examples are shown which have the purpose of better illustrating the compositions and methods detected in the present invention, such examples are not to be considered in any way as a limitation of the preceding description and of the subsequent claims.

### EXEM PLES

### Introduction

The purpose of the studies performed by the authors of the present invention and illustrated hereinafter is to apply the performic acid (PFA, CAS 107-32-4) as biocide even in industrial cooling systems (for example *"cooling towers"),* a well different type of plants, in terms of quality of water to be treated and of the used operating conditions, with respect to the civil depuration plants in which PFA is already used as biocide.

The need for dosing specific biocides in the *Cooling Tower* systems arises from the fact that the microorganisms grow and multiply in such systems, by creating problems of corrosion, *biofouling,* generation of biofilm and safety.

The biocidal products generally used in such field are divided into three macro-classes:
- Not oxidizing biocides
- Oxidizing biocides
- Bio-dispersants

PFA, such as the sodium hypochlorite and the peracetic acid, is an oxidizing biocide. PFA is instable and tends to degrade even at low temperatures. Due to these features thereof, PFA has to be necessarily generated *in situ* and subsequently dosed. The scheme 1 shown hereinafter illustrates the main reactions of PFA.

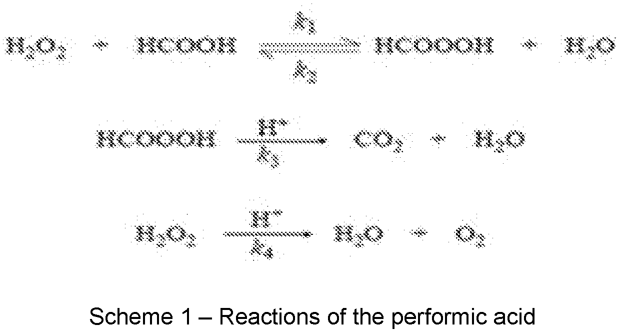

In particular, while having an acid constant comparable to PAA (peracetic acid CAS 79-21-0), PFA would seem not to influence the pH of water in the considered systems. It is possible to assume that this is due to the quick degradation of PFA to formic acid (FA), which subsequently decomposes to carbon dioxide and water, thus not influencing the pH of the system. On the contrary, in case of PAA, acetic acid is generated, which influences the pH of the system. On the contrary, the sodium hypochlorite is widely used in the *Cooling* Tower systems with due care to avoid unwished problems of *"pitting"* and formation of reaction products such as the chlorine amines.

As mentioned, PFA is generally used as biocide in industrial or civil waste water the quality thereof results to be extremely different with respect to the water present in an industrial cooling system.

In Italy, the Decree of the Regional Government Nr. 1053/2003 defines:
- the regulation of waste and the regime authorizing the household and assimilated waste water, the urban waste water deriving from agglomerates with population lower than 2,000 PE, as well as the discharges of dangerous substances;
- the type and technical characterization of the individual systems for the treatment to be applied to the settlements, installations, buildings/isolated nuclei which discharge household waste water in receptors different from the sewage system;
- the type of treatment to be applied to the discharges deriving from the different categories of agglomerates and the emission limit values;
- Emission limit values in surface water and in sewage system.

The monitored pathogens result to be even *Enterococci, E.Coli* and *Faecal coliformus.*

As far as the water present in a cooling tower is concerned, the monitored parameters are pH, the total aerobic and anaerobic microbial load (by NACE TM 0194-2004, ASTM D4412 (2002), EASY CULT *method)* and all analytes which can provide information about phenomena of corrosion, deposit and trend of the chemical treatment. In a cooling tower the pathogen which is monitored is mainly *Legionella pneumophila.* The feed water used in the cooling towers (MU) usually comes from river.

For managing the system, it results to be important to monitor parameters such as Hardness, the sulphates, the silicates (deposition monitoring and prevention), the chlorides, the pH, the conductivity (corrosion management and prevention) which in the "cooling tower" system tend to concentrate.

### Example 1 - Microbial regrowth test in water deriving from cooling systems

The authors of the present invention tested the PFA capability of hindering phenomena of microbial *regrowth* due to the reaction products of the same PFA in water deriving from the basin of a cooling tower.

In the performed tests a solution of 85% formic acid was used as source of formic acid and water sampled from the collection basin of a cooling tower as "white".

The hydrogen peroxide used in the tests has a concentration of 30%. The temperature of the tests is 30-40°C.

The monitoring methods used to monitor the regrowth phenomenon are the following:
NACE TM 0194-2004, ASTM D4412 (2002) and EASY CULT method.

### Example 1a

A first *regrowth* test used the following reagents:
- White: water collected on 28/04 from cooling tower collection basin;
- Solution 1: White + 10 mg/L Formic Acid Solution;
- Solution 2: White + 30 mg/L Formic Acid Solution.

EasyCult kits were used, generally intended to monitor the microbiological contaminations in industrial environments. The contact time of the AF solutions before inoculation with the tested microbial species was equal to 30 minutes.

Results: after 48 hours of exposure to the above-mentioned solutions, all tested microbial samples showed a load of 10⁶ UFC which corresponds to the higher limit of the EasyCults used for the test.

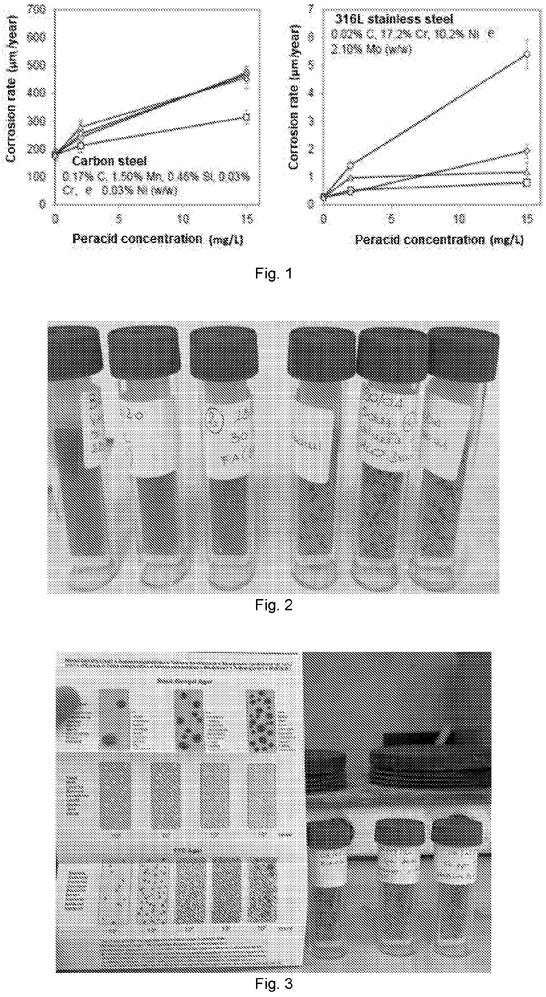

After 5 days, all three steaks lead back to the same reading of microbial load of 10⁶. The reading after 5 days shows a decrease in bacterial load, the same both for the white and for the solutions in which different concentrations of formic acid were added; this parameter decreases, by demonstrating that the regrowth phenomenon does not appear. The results of this test, obtained on 28/04 and 30/40 in diluted water, are illustrated in Figure 2.

### Example 1b

A second *regrowth* test used the following reagents:
- White: water collected on 06/05 from cooling tower collection basin;
- Solution 1: White + 10 mg/L Formic Acid Solution;
- Solution 2: White + 50 mg/L Formic Acid Solution.

Contact time of AF solutions before inoculation: 30 minutes.

Before inoculation, all samples were diluted with demineralized water in ratio 1:100.

Results: the microbial load in the samples tested after 48 hours resulted to be comparable in the three samples, that is equal to 10⁶ (let's remind the dilution 1:100 - Figure 3). It was then possible to reconfirm the microbial load detected in the water used during the tests shown in the example 1a, in Avril, as well as to confirm that the formic acid would not seem to perform biocidal effect. On 7 May the solutions, after dilution, were inoculated for the second part of test. After 48 hours *regrowth* phenomena were not observed and the two additivated samples performed a microbial load comparable to the white, that is equal to 10⁶. The results of this test were illustrated in Figure 4.

### Example 1c

Differently from the previously illustrated tests, in this case even the hydrogen peroxide was introduced in the solutions, substance in equilibrium with the formic acid in the system for producing performic acid. Moreover, in order to improve the sensitivity of the results, the tests were performed by using *vials* following the NACE TM 0194-2004 method for aerobic microbial loads (salinity 2000 ppm). The contact time of the AF solutions before inoculation was equal to 30 minutes. The number of vials considered for each test was N=8.

This test then used the following solutions:
- White: water collected on 13/07 from cooling tower collection basin and diluted 1 to 30 with demineralized water
- Solution 1: diluted White + 30 mg/L Formic Acid Solution;
- Solution 2: diluted White + 10 mg/L Formic Acid Solution;
- Solution 3: diluted White + 30 mg/L Formic Acid Solution + 23.4 mg/L H₂O₂;
- Solution 4: diluted White + 10 mg/L Formic Acid Solution + 7.8 mg/L H₂O₂.

Results: the results of the performed test are shown in Figure 5. These results did not reveal any regrowth phenomena.

### Example 1d

An additional test was performed aiming at verifying if the formate ion could act as feed for bacteria.

The formate ion was obtained through salification of the formic acid with 30% NaOH (sodium hydroxide). The pH is 7 and this favours the presence of the formate ion in solution.

The following reagents were used:
- White: water collected on 13/07 from cooling tower collection basin diluted 1 to 30 with demineralized water;
- Solution 2: White + 30 mg/L Salified Formic Acid Solution + 23.4 mg/L H₂O₂.

The contact time of the AF solutions before inoculation was equal to 30 minutes. The number of vials considered for each test was N= 8.

Results: the results of this test are shown in Figure 6. Even under these conditions no regrowth phenomena were revealed.

### Example 2 - Comparative tests between PFA, peracetic acid (PAA) and perpropionic acid (PPA)

The literature data and laboratory tests performed by the authors of the present invention revealed the following PFA properties.

The disinfection effectiveness determined for the considered peracids is shown hereinafter in increasing order:
for *E. coli:* $PFA > PAA$ for enterococci: $PFA > PPA$

The authors of the invention found that PFA performs a better biocidal/disinfecting action than the biocides with which it was compared. In particular, PFA demonstrated to be capable of reducing by more than 99% the UFC/ml of *Legionella* at lower dosages than the peracetic acid (PAA). PFA resulted to have, at lower dosages and with shorter contact time, a higher effectiveness of PAA and of the chlorinated biocides. The PFA decomposition rate makes this substance much more effective than PAA; at the same time, the use of PFA would seem not to have produced pH variations of treated water.

**Table 2: Effect of performic acid on viability of L. pneumophila after disinfection with 0, 1, 2 and 4 mg of PFA/L by using a contact time of 10 minutes. The detection limit was equal to 10 cfu/mL.**

| Concentration, mg PFA/L | Bacterial density, cfu/mL | | Reduction % |
|---|---|---|---|
| | Average | Range | |
| 0 mg/L | 6.3 x 10⁴ | 4.4x10⁴ - 7.1x10⁴ | - |
| 1 mg/L | 1.3 x 10² | 1.1x10² - 1.4x10² | 99.8 |
| 2 mg/L | Nd¹ | Nd¹ | 100 |
| 4 mg/L | Nd¹ | Nd¹ | 100 |

| | | | |
|---|---|---|---|
| ¹Nd = not detected | | | |

### Conclusions

The microbial regrowth tests performed in laboratory by the authors of the present invention demonstrated that both PFA and its decomposition products, such as the formate ion and the formic acid, do not behave as nutrients in the cooling systems and do not favour the bacterial growth in the treated system.

PFA performance decreases upon increasing pH; however, in a cooling tower system often this parameter is controlled and kept in a pre-established range by using acids or bases. The pH of the water processed in a cooling tower if controlled, is kept in the range 6.5-8 then comparable to that of the water present in a civil waste water plant.

The metal portions in a cooling tower have a carbon steel metallurgy such as C₁₀₁₈, C₁₀₁₀, AISI 316 L then not particularly resistant to phenomena of corrosion due to acids. This because the corrosion often is a less relevant problem than the formation of deposit due to calcium carbonate the presence thereof increases in the system after the concentration to which the water is subjected in tower and the temperatures in the system. PFA, as it is an acid, could be not recommended in a cooling system, but it was checked that it does not provide high corrosion rates on AISI 316 L metallurgy whereas on carbon steel metallurgy the corrosion rates result to be higher than PAA, but however lower than 500 um/y (Figure 1).

Measurements and literature data of TOC, BOD₅ and COD show a poor persistence in the system of the PFA reaction products. The data found in literature and the performed *regrowth* tests then allowed to select PFA as biocide for cooling towers and/or cooling circuits in general.

## Claims

1. A use of a composition comprising performic acid (PFA) as biocide in an industrial cooling system and/or to hinder the regrowth of microorganisms in an industrial cooling system, wherein said composition comprises a concentration of PFA comprised between 0.5 and 0.8 ppm, and wherein said composition further comprises hydrogen peroxide.

2. The use according to claim 1, wherein said composition comprises PFA and hydrogen peroxide in a molar ratio comprised between 10:1 a 1:10.

3. The use according to any one of claims 1 or 2, wherein said composition is a solution in equilibrium further comprising formic acid and hydrogen peroxide, preferably wherein said formic acid is present in an amount comprised between 2 and 25% by weight with respect to the total weight of the composition and the molar ratio between PFA and hydrogen peroxide is comprised between 10:1 and 1:10.

4. The use according to any one of claims 1 to 3, wherein said composition prevents said microorganisms from regrowing in said cooling system within the subsequent 12-48 hours.

5. The use according to any one of claims 1 to 4, wherein said microorganisms comprise Gram-positive and/or Gram-negative bacteria, fungi, viruses, preferably wherein said Gram-positive and/or Gram-negative bacteria comprise microorganisms belonging to *Enterococcus, Escherichia, Legionella, Klebsiella, Citrobacter* genus, preferably they comprise Escherichia coli and Legionella pneumophila species.

6. The use according to any one of claims 1 to 5, wherein said industrial cooling system comprises cooling water.

7. The use according to the preceding claim, wherein said cooling water is **characterized by** an amount of dissolved oxygen comprised between 2 and 20 ppm.

8. A method for preventing, reducing or removing and/or for hindering the regrowth of microorganisms in an industrial cooling system, comprising at least the following step of:
a) putting in contact said cooling system with a composition comprising performic acid (PFA) in an amount effective as biocide and/or to hinder the regrowth of said microorganisms, wherein said composition comprises PFA in a concentration between 0.5 and 0.8 ppm and wherein said PFA is generated *in situ* starting from formic acid and hydrogen peroxide.

9. The method according to claim 8, wherein said step a) has a duration at least equal to 30 minutes and/or it is performed at a temperature comprised between 5 and 50°C.

## Patentansprüche

1. Verwendung einer Zusammensetzung, umfassend Perameisensäure (PFA) als Biozid in einem industriellen Kühlsystem und/oder um das Nachwachsen von Mikroorganismen in einem industriellen Kühlsystem zu hemmen, wobei die Zusammensetzung eine Konzentration an PFA umfasst, die zwischen 0,5 und 0,8 ppm umfasst ist, und wobei die Zusammensetzung ferner Wasserstoffperoxid umfasst.

2. Verwendung nach Anspruch 1, wobei die Zusammensetzung PFA und Wasserstoffperoxid in einem Molverhältnis umfasst, das zwischen 10 : 1 und 1 : 10 umfasst ist.

3. Verwendung nach einem der Ansprüche 1 oder 2, wobei die Zusammensetzung eine Gleichgewichtslösung ist, ferner umfassend Ameisensäure und Wasserstoffperoxid, vorzugsweise wobei die Ameisensäure in einer Menge, die zwischen 2 und 25 Gew.-% bezogen auf das Gesamtgewicht der Zusammensetzung umfasst, vorliegt und das Molverhältnis zwischen PFA und Wasserstoffperoxid zwischen 10 : 1 und 1 : 10 umfasst ist.

4. Verwendung nach einem der Ansprüche 1 bis 3, wobei die Zusammensetzung verhindert, dass die Mikroorganismen in dem Kühlsystem innerhalb der nachfolgenden 12-48 Stunden nachwachsen.

5. Verwendung nach einem der Ansprüche 1 bis 4, wobei die Mikroorganismen grampositive und/oder gramnegative Bakterien, Pilze, Viren umfassen, vorzugsweise wobei die grampositiven und/oder die gramnegativen Bakterien Mikroorganismen umfassen, die zu einer *Enterococcus-, Escherichia-, Legionella-, Klebsiella-, Citrobacter-*Gattung gehören, vorzugsweise wobei sie Escherichia-coli- und Legionella-pneumophila-Arten umfassen.

6. Verwendung nach einem der Ansprüche 1 bis 5, wobei das industrielle Kühlsystem Kühlwasser umfasst.

7. Verwendung nach dem vorstehenden Anspruch, wobei das Kühlwasser **gekennzeichnet ist durch** eine Menge an gelöstem Sauerstoff, die zwischen 2 und 20 ppm umfasst ist.

8. Verfahren zum Verhindern, Reduzieren oder Entfernen und/oder zum Hemmen des Nachwachsens von Mikroorganismen in einem industriellen Kühlsystem, umfassend mindestens den folgenden Schritt: a) Inberührungbringen des Kühlsystems mit einer Zusammensetzung, umfassend Perameisensäure (PFA) in einer Menge, die als Biozid wirksam ist und/oder um das Nachwachsen der Mikroorganismen zu hemmen, wobei die Zusammensetzung PFA in einer Konzentration zwischen 0,5 und 0,8 ppm umfasst und wobei die PFA ausgehend von Ameisensäure und Wasserstoffperoxid *in situ* erzeugt wird.

9. Verfahren nach Anspruch 8, wobei der Schritt a) eine Dauer, die mindestens gleich 30 Minuten ist, aufweist und/oder er bei einer Temperatur, die zwischen 5 und 50 °C umfasst ist, durchgeführt wird.

## Revendications

1. Utilisation d'une composition comprenant de l'acide performique (PFA) comme biocide dans un système de refroidissement industriel et/ou pour entraver la repousse de micro-organismes dans un système de refroidissement industriel, dans laquelle ladite composition comprend une concentration en PFA comprise entre 0,5 et 0,8 ppm, et dans laquelle ladite composition comprend en outre du peroxyde d'hydrogène.

2. Utilisation selon la revendication 1, dans laquelle ladite composition comprend du PFA et du peroxyde d'hydrogène dans un rapport molaire compris entre 10:1 et 1:10.

3. Utilisation selon l'une quelconque des revendications 1 ou 2, dans laquelle ladite composition est une solution en équilibre comprenant en outre de l'acide formique et du peroxyde d'hydrogène, de préférence dans laquelle ledit acide formique est présent en une quantité comprise entre 2 et 25 % en poids par rapport au poids total de la composition et au rapport molaire entre PFA et le peroxyde d'hydrogène est compris entre 10:1 et 1:10.

4. Utilisation selon l'une quelconque des revendications 1 à 3, dans laquelle ladite composition empêche lesdits micro-organismes de repousser dans ledit système de refroidissement au cours des 12 à 48 heures suivantes.

5. Utilisation selon l'une quelconque des revendications 1 à 4, dans laquelle lesdits micro-organismes comprennent des bactéries à Gram positif et/ou à Gram négatif, des champignons, des virus, de préférence dans laquelle lesdites bactéries à Gram positif et/ou à Gram négatif comprennent des micro-organismes appartenant *aux genres Enterococcus, Escherichia, Legionella, Klebsiella, Citrobacter*, comprennent de préférence les espèces Escherichia coli et Legionella pneumophila.

6. Utilisation selon l'une quelconque des revendications 1 à 5, dans laquelle ledit système de refroidissement industriel comprend de l'eau de refroidissement.

7. Utilisation selon la revendication précédente, dans laquelle ladite eau de refroidissement est **caractérisée par** une quantité d'oxygène dissous comprise entre 2 et 20 ppm.

8. Procédé permettant de prévenir, réduire ou éliminer et/ou entraver la repousse de micro-organismes dans un système de refroidissement industriel, comprenant au moins l'étape suivante consistant à : a) mettre en contact ledit système de refroidissement avec une composition comprenant de l'acide performique (PFA) dans une quantité efficace tel que biocide et/ou pour entraver la repousse desdits micro-organismes, dans laquelle ladite composition comprend du PFA à une concentration comprise entre 0,5 et 0,8 ppm et dans laquelle ledit PFA est généré *in situ* à partir d'acide formique et de peroxyde d'hydrogène.

9. Procédé selon la revendication 8, dans lequel ladite étape a) a une durée au moins égale à 30 minutes et/ou elle est effectuée à une température comprise entre 5 et 50 °C.
